# EUROPEAN PATENT APPLICATION

(11) **EP 3 020 349 A1**
(43) Date of publication of application: **18.05.2016**
(21) Application number: 15194280.2
(22) Date of filing: 12.11.2015
(51) Int. Cl.: A61B 17/70

(54) **GROWING SPINAL RODS AND PEDICLE SCREWS FOR SECURING MULTIPLE SPINAL RODS**

(30) Priority: 14.11.2014 US 201462079609 P
(71) Applicant: K2M, Inc., Leesburg, VA 20175 (US)
(72) Inventor: Mehdian, Hossein, Nottingham, NG7 2GZ (GB)
(74) Representative: Hoffmann Eitle

(57) **Abstract**

An adjustable spinal rod includes a first section and a second section. The first section has a first base portion and a first coupling portion that extends from the first base portion. The second section has a second base portion and a second coupling portion extending from the second base portion. The second coupling portion is axially aligned and slidably engaged with the first coupling portion. The adjustable spinal rod has a first configuration where the adjustable spinal rod defines a first length and a second configuration where the adjustable spinal rod defines a second length different from the first length.

## Description

### Cross-Reference to Related Application

This application claims the benefit of, and priority to, U.S. Provisional Patent Application Serial No. 62/079,609, filed on November 14, 2014, the entire contents of which are hereby incorporated by reference.

### Background

### 1. Technical Field

The present disclosure relates to orthopedic surgical devices for stabilizing and fixing the bones and joints of a body. Specifically, the present disclosure relates to growing spinal rods that expand with vertebral bodies of a spinal column. In addition, the present disclosure relates to pedicle screws that are securable to multiple spinal rods.

### 2. Discussion of Related Art

The spinal column is a complex system of bones and connective tissues that provide support for the human body and protection for the spinal cord and nerves. The human spine is comprised of thirty-three vertebrae at birth and twenty-four as a mature adult. Between each pair of vertebrae is an intervertebral disc, which maintains the space between adjacent vertebrae and acts as a cushion under compressive, bending, and rotational loads and motions.

There are various disorders, diseases, and types of injury that the spinal column may experience in a lifetime. The problems may include, but are not limited to, scoliosis, kyphosis, excessive lordosis, spondylolisthesis, slipped or ruptured disc, degenerative disc disease, vertebral body fracture, and tumors. Persons suffering from any of the above conditions typically experience extreme and/or debilitating pain, and often times diminished nerve function.

Conventionally, orthopedic surgeons receive training in the use of orthopedic devices and the performance of surgical methods to correct vertebral column injuries and diseases by the application of methods and devices on cadavers. The amount of training for each surgeon is limited by the expense, availability, scheduling, and other logistic requirements associated with the use of cadavers.

Further, spine surgeons, when planning for a surgical procedure on a specific patient, are normally limited to a study of two-dimensional radiographic data and a complete lack of hands-on manipulation rehearsal of a method prior to operating on a patient. In recent years there has been a growing number of orthopedic practices and hospitals that have made the transition from film to all digital environments. Software based tools for orthopedic image review, analysis, and preoperative planning are becoming conventional tools of the orthopedic surgeon. While advances in surgical planning have been made, they are simply limited to improvements in providing two-dimensional data for study and planning. To receive hands-on training or to rehearse a surgical method, a surgeon is still limited to the use of cadavers.

With such training and rehearsal limitations, it is not uncommon during an actual surgical procedure for a surgeon to encounter unforeseen anatomical or biomechanical conditions that may require an immediate revision of the surgical plan as it proceeds. The need to provide more, less expensive ways to train surgeons or to permit hands-on surgery planning and rehearsal in the use of spinal surgery methods and devices is particularly needed in the treatment of spine conditions, such as scoliosis. It is not uncommon in the surgical treatment of scoliosis that forceful manipulation and realignment of the spinal column can be a long, complicated mechanical effort that often includes a serious threat of damage to the spinal cord.

Further, the biomechanical behavior and particularly soft tissue forces on the spinal column when applying methods and devices to a cadaver are far different from that which are normally experienced in a surgical procedure on a living patient.

Thus, a need exists for a three-dimensional hands-on spinal surgery modeling system that can be used by surgeons for training in the use of devices and methods, and that can also be used in the planning and manual rehearsal of surgical procedures for patients. In addition, there is need for adjustable or growing spinal rods to be used for training or during surgical procedures. Moreover, there is a need for pedicle screws that can secure multiple spinal rods during training or surgical procedures.

### Summary

In an aspect of the present disclosure, an adjustable spinal rod includes a first section and a second section. The first section has a first base portion and a first coupling portion that extends from the first base portion. The second section has a second base portion and a second coupling portion extending from the second base portion. The second coupling portion is axially aligned and slidably engaged with the first coupling portion. The adjustable spinal rod has a first configuration where the adjustable spinal rod defines a first length and a second configuration where the adjustable spinal rod defines a second length different from the first length.

In aspects, the first and second base portions each define a circular cross-section. The first coupling portion may include a first finger and the second coupling portion may include a second finger. The first and second base portions may each define a semi-circular cross-section and be slidably engaged with one another.

In some aspects, the adjustable spinal rod includes a first coupling member disposed about the first and second fingers to slidingly couple the first and second sections together. The first finger may include a pair of ribs that define a first groove therebetween. A portion of the first coupling member may be positionable in the first groove about the first and second fingers. The second finger may include a pair of ribs that define a second groove therebetween. A portion of the second coupling member may be positionable within the second groove about the first and second fingers.

In certain aspects, the first section has a first diameter and the first coupling portion defines a blind hole having a second diameter about a longitudinal axis of the first section. The second base portion may have a diameter equal to the first diameter of the first base portion and the second coupling portion may have a third diameter less than the second diameter of the blind hole. The second coupling portion may be slidingly receivable within the blind hole of the first coupling portion.

In particular aspects, the adjustable spinal rod includes a coupling member that is disposable about the first coupling portion to fix the first and second coupling portions relative to one another. The coupling member may be disposed about the first and second coupling portions. The second coupling portion may be frictionally engaged and secured within the blind hole of the first coupling portion.

In aspects, the first and second sections defines a curved longitudinal axis of the adjustable spinal rod.

In another aspect of the present disclosure, a spinal construct includes first and second adjustable spinal rods and a pedicle screw. The first adjustable spinal rod defines a first longitudinal axis and the second adjustable spinal rod defines a second longitudinal axis that is parallel with the first longitudinal axis. Each of the first and second adjustable spinal rods includes a first section and a second section. The first sections have a first base portion and a first coupling portion that extends from the first base portion. The second sections have a second base portion and a second coupling portion that extends from the second base portion. The second coupling portions are axially aligned and slidably engaged with the first coupling portion of the respective adjustable spinal rod. The first and second adjustable spinal rods each have a first configuration where the adjustable spinal rod defines a first length and a second configuration where the adjustable spinal rod defines a second length different from the first length. The pedicle screw includes a head and a shank. The shank is configured to secure the pedicle screw to a bony element. The head is disposed at one end of the shank and receives the first base portion of the first adjustable spinal rod and the first base portion of the second adjustable spinal rod to fix the first and second adjustable spinal rods relative to one another.

In aspects, the head may define a first saddle and a second saddle. The first saddle receives the first base portion of the first adjustable spinal rod to fix the first spinal rod to the pedicle screw. The second saddle may receive the first base portion of the second adjustable spinal rod to fix the second spinal rod to the pedicle screw. The first and second saddle may be configured to independently fix the first and second spinal rods to the pedicle screw.

In some aspects, the shank defines a longitudinal shank axis. The first saddle may define a first saddle axis that is offset from the shank axis by a first angle and the second saddle may define a second saddle axis that is offset from the shank axis by a second angle. The first and second angles may be defined in a common plane. The first and second angles may be defined in different directions from the shank axis. The first and second angles may be equal to one another. The first angle may be in a range of about 15° to about 90°. Alternatively, the first angle may be about 0° and the second angle may be about 90°.

In certain aspects, the head includes sidewalls that define a channel. The channel may receive the first base portion of the first adjustable spinal rod and the first base portion of the second adjustable spinal rod. The sidewalls may cooperate to define an opening. The pedicle screw may include a set screw that is received within the opening to secure the first base portions of the first and second adjustable spinal rods within the channel.

In particular aspects, the head includes bottom surfaces that oppose the opening and define a portion of the channel. The bottom surfaces may include a divider that engages the first base portions of the first and second adjustable spinal rods when the first base portions are received within the channel. The divider may be aligned with a longitudinal shank axis of the shank.

In another aspect of the present disclosure, a pedicle screw includes a shank and a head. The shank is configured to secure the pedicle screw to a bony element. The head is disposed at one end of the shank and defines a first saddle and a second saddle. The first saddle is configured to receive a portion of a first spinal rod to fix the first spinal rod to the pedicle screw. The second saddle is configured to receive a portion of a second spinal rod to fix the second spinal rod to the pedicle screw. The first and second saddles are configured to independently fix the first and second spinal rods to the pedicle screw.

In aspects, the shank may define a longitudinal shank axis. The first saddle may define a first saddle axis that is offset from the shank axis by a first angle and the second saddle may define a second saddle axis that is offset from the shank axis by a second angle. The first and second angles may be defined in a common plane.

In some aspects, the first and second angles are defined in different directions from the shank axis. The first and second angles may be equal to one another. The first angle may be in a range of about 15° to about 90°. Alternatively, the first angle may be about 0° and the second angle may be about 90°.

In another aspect of the present disclosure, a pedicle screw includes a shank, a head, and a set screw. The shank is configured to secure the pedicle screw to a bony element. The head may be disposed at one end of the shank and has sidewalls that define a channel. The channel is configured to receive a portion of a first spinal rod and a portion of a second spinal rod. The sidewalls cooperate to define an opening. The set screw is receivable within the opening to secure the portions of the first and second spinal rods within the channel.

In aspects, the head includes bottom surfaces that oppose the opening and define a portion of the channel. The bottom surfaces may include a divider that is configured to engage the portions of the first and second spinal rods that are received within the channel. The divider may be aligned with a longitudinal shank axis of the shank.

In another aspect of the present disclosure, an adjustable spinal rod includes a first section, a second section, and coupling members. The first section defines a longitudinal axis and includes a first base portion and a first finger. The first finger has a pair of ribs that define a first groove. The second section includes a second base portion and a second finger. The second finger extends towards the first section and has a pair of ribs that define a second groove. The second finger is slidable against the first finger along the longitudinal axis. The coupling members are positioned about the first and second fingers to slidably couple the first and second sections together. Each of the coupling members is disposed within one of the first or second grooves. The adjustable spinal rod has a first configuration such that the adjustable spinal rod defines a first length and a second configuration such that the adjustable spinal rod defines a second length that is different from the first length.

In another aspect of the present disclosure, an adjustable spinal rod includes first and second sections. The first section has a first diameter and defines a blind hole along a longitudinal axis having a second diameter. The second section includes a shaft that extends towards the first section. The shaft has a shaft diameter that is less than the second diameter such that the shaft is slidably received within the blind hole The adjustable spinal rod has a first configuration such that the adjustable spinal rod defines a first length and a second configuration such that the adjustable spinal rod defines a second length that is different from the first length.

In another aspect of the present disclosure, a pedicle screw for securing multiple spinal rods to a vertebral body includes a shank and a head. The shank is configured to secure the pedicle screw to the vertebral body and defines a shank axis. The head has first and second saddles. Each of the saddles is configured to secure a respective spinal rod to the head. The first saddle defines a first saddle axis that is offset from the shank axis by a first angle and the second saddle defines a second saddle axis that is offset from the shank axis by a second angle. The first and second angles are defined in a common plane and are in different directions from the shank axis.

In another aspect of the present disclosure, a pedicle screw for securing multiple spinal rods to a vertebral body includes a shank, a head, and a set screw. The shank is configured to secure the pedicle screw to the vertebral body. The head has sidewalls that define an opening and a channel. The channel is dimensioned to receive at least two spinal rods therethrough. The set screw is threadably inserted through the opening and is configured to engage each of the at least two spinal rods to secure each of the spinal rods to the head.

In another aspect of the present disclosure, a spinal construct for aligning vertebral bodies of a spine of a patient or a spine model includes at least one adjustable spinal rod and at least one pedicle screw. The at least one spinal rod may be any of the adjustable spinal rods disclosed herein and the at least one pedicle screw may be any of the pedicle screws disclosed herein.

Further, to the extent consistent, any of the aspects described herein may be used in conjunction with any or all of the other aspects described herein.

### Brief Description of the Drawings

Various aspects of the present disclosure are described hereinbelow with reference to the drawings, which are incorporated in and constitute a part of this specification, wherein:
FIG. 1 is a front, perspective view of a spinal surgery modeling system provide in accordance with the present disclosure;
FIG. 2 is a back, perspective view of the spinal surgery modeling system of FIG. 1;
FIG. 3 is a perspective view of a spinal construct provided in accordance with the present disclosure in a first configuration;
FIG. 4 is a perspective view of the spinal construct of FIG. 3 in a second configuration;
FIG. 5 is a perspective view of the spinal construct of FIG. 3 in a third configuration;
FIG. 6 is an enlarged view of the indicated area of detail of FIG. 5;
FIG. 7 is a perspective view of another spinal construct provided in accordance with the present disclosure;
FIG. 8 is a perspective view of yet another spinal construct provided in accordance with the present disclosure secured to a spine of a patient;
FIG. 9 is an enlarged view of the indicated area of detail of FIG. 8;
FIG. 10 is a perspective view of another spinal construct provided in accordance with the present disclosure in a first configuration;
FIG. 11 is a perspective view of the spinal construct of FIG. 10 in a second configuration;
FIG. 12 is a side longitudinal cross-sectional view of the spinal construct of FIG. 11;
FIG. 13 is a perspective view of a spinal construct provided in accordance with the present disclosure secured to a spine of a patient;
FIG. 14 is a perspective view of a pedicle screw provided in accordance with the present disclosure;
FIG. 15 is a front view of the pedicle screw of FIG. 14;
FIG. 16 is a side view of the pedicle screw of FIG. 14;
FIG. 17 is a perspective view of the pedicle screw of FIG. 14 secured to two spinal rods;
FIG. 18 is a front view of the pedicle screw of FIG. 17;
FIG. 19 is a perspective view of another pedicle screw provided in accordance with the present disclosure;
FIG. 20 is a front view of the pedicle screw of FIG. 19;
FIG. 21 is a perspective view of the pedicle screw of FIG. 19 secured to two spinal rods;
FIG. 22 is a front view of the pedicle screw of FIG. 21;
FIG. 23 is a posterior view of pedicle screws of FIG. 14 secured to a spine of a patient and two spinal rods; and
FIG. 24 is a side view of the pedicle screws of FIG. 23.

### Detailed Description

Embodiments of the present disclosure are now described in detail with reference to the drawings in which like reference numerals designate identical or corresponding elements in each of the several views. As used herein, the term "clinician" refers to a doctor, a nurse, or any other care provider and may include support personnel. Throughout this description, the term "proximal" will refer to the portion of the device or component thereof that is closest to the clinician and the term "distal" will refer to the portion of the device or component thereof that is farthest from the clinician. In addition, the term "cephalad" is used in this application to indicate a direction toward a patient's head, whereas the term "caudad" indicates a direction toward the patient's feet. Further still, for the purposes of this application, the term "lateral" indicates a direction toward a side of the body of the patient, i.e., away from the middle of the body of the patient. The term "posterior" indicates a direction toward the patient's back, and the term "anterior" indicates a direction toward the patient's front. Additionally, in the drawings and in the description that follows, terms such as front, rear, upper, lower, top, bottom, and similar directional terms are used simply for convenience of description and are not intended to limit the disclosure.

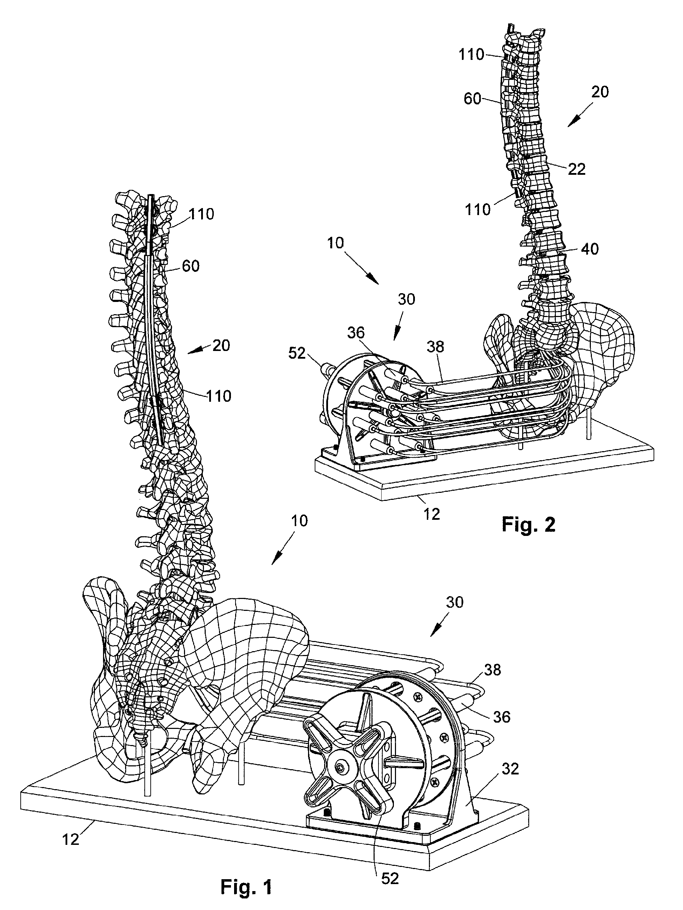

As shown in FIGS. 1 and 2, a spinal surgery modeling system 10 includes a spine model 20, a spine movement device 30, and optionally, one or more spinal constructs (e.g., adjustable or growing rod 60 and pedicle screws 110). The spine model 20 and the spine movement device 30 may each be removably or fixedly attached to a base 12, and the adjustable rod 60 and screw 70 may be affixed to the spine model 20. The spine model 20 may be a cadaveric or synthetic anatomically and mechanically correct spine model of a pediatric, adult, or geriatric spine which may exhibit a variety of spine pathologies.

The spine movement device 30 includes a main frame 32, cylinders 36, and a handle 52. Each of the cylinders 36 is fluidly connected by tubing 38 to inflation members 40 of the spinal model 20. As the handle 52 is rotated in a first direction, fluid (not shown) is displaced from the cylinders 36, through the tubing 38, and into the inflation members 40 to hydraulically inflate or expand the inflation members 40. As the handle 52 is rotated in a second direction opposite the first direction, fluid (not shown) is drawn into the cylinders 36 from the inflation members 40 through the tubing 38 to hydraulically deflate or contract the inflation members 40. The fluid may be a liquid or a gas. The amount of fluid contained within one of the cylinders 36 may be the same or different from the amount of fluid contained within another one of the cylinders 36.

The inflation members 40 may be positioned within a disc space between each of the vertebral bodies 22 of the spinal model such that as the handle 52 is rotated in the first direction, the inflation members 40 are inflated to expand the disc space between the vertebral bodies 22 to simulate growth of a spine with the spinal model 20. For a detailed description of an exemplary spine model and spine movement device reference may be made to commonly owned U.S. Patent Application Serial No. 14/798,591, filed July 14, 2015, the entire contents of which are hereby incorporated by reference.

The vertebral bodies 22 of the spine model 20 are freely accessible to a clinician using the system 10. Accordingly, various spinal constructs, such as an adjustable rod 60 and pedicle screws 110, may be placed on the vertebral bodies 22 to allow the clinician to practice methods of placing such spinal constructs on a spine. In addition, the clinician may observe the interaction of the spinal constructs with a spine. For example, the clinician may observe the interaction of the spinal constructs with a spine as the spine grows by rotating the handle 52 in the first direction as detailed above.

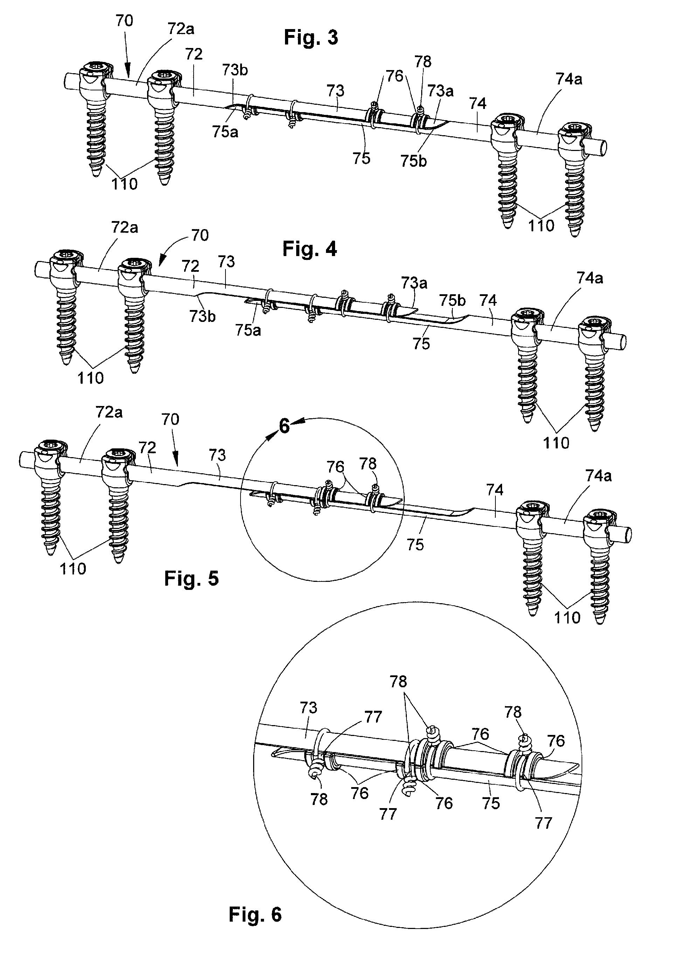

With reference to FIGS. 3-6, an adjustable spinal rod 70 is provided in accordance with the present disclosure and includes a first section 72, a second section 74, and coupling members 78. The adjustable spinal rod 70 may be substituted for the adjustable rod 60 in spinal surgery modeling system 10. The spinal rod 70 may be provided with one or more pedicle screws 110 secured to the first and second sections 72, 74 to secure the spinal rod 70 to a spine model (e.g., spine model 20) or a spine of a patient as detailed below.

The first section 72 has a base portion 72a and a finger 73 that extends towards the second section 74. The second section 74 has a base portion 74a and a finger 75 that extends towards the first section 72. The base portions 72a, 74a define a substantially cylindrical shape along a longitudinal axis having a diameter substantially equal to one another. The fingers 73, 75 are slidable relative to one another and are shaped such that the fingers 73, 75 together form a substantially cylindrical shape having a diameter substantially equal to the diameter of the base portions 72a, 74a. Each finger 73, 75 includes ribs 76 extending radially from an outer surface. With particular reference to FIG. 6, the ribs 76 of each finger 73, 75 are disposed in pairs that define a groove 77 between each pair of ribs 76. Each finger 73, 75 has a tip 73a, 75a and an end surface 73b, 75b. The tips 73a, 75a and end surfaces 73b, 75b may be shaped to form a smooth transition between the first and second sections 72, 74 when the tip 73a abuts the end surface 75b and the tip 75a abuts the end surface 73b.

A coupling member 78 is wrapped about each finger 73, 75 and is disposed in one of the grooves 77 to slidably secure the first and second sections 72, 74 together. As shown, the coupling member 78 is a wire that is wrapped around each finger 73, 75 and twisted together to slidably secure the first and second sections 72, 74 together. It will be appreciated that the ribs 76 longitudinally fix the coupling member 78 relative to the respective finger 73, 75. As shown, each finger 73, 75 has two pairs of ribs 76 defining two grooves 77; however, it is contemplated that each finger 73, 75 may have a greater or few number of ribs 76. For example, each finger 73, 75 may have three ribs 76 that define two grooves 77.

It is within the scope of this disclosure that one finger 73, 75 may have a number of ribs 76 and grooves 77 different from the other finger 73, 75. For example, one finger (e.g., finger 73) may have two pairs of ribs 76 defining two grooves 77 and the other finger (e.g., finger 75) may have a single pair of ribs 76 defining a single groove 77.

The first and second sections 72, 74 are slidable relative to one another between a compressed or first configuration (FIG. 3), an intermediate or second configuration (FIG. 4), and an extended or third configuration (FIG. 5) to permit adjustment of a length of the spinal rod 70. In the first configuration, the tip 73a of the finger 73 of the first section 72 abuts the end surface 75b of the finger 75 of the second section 74 and a tip 75a of the finger 75 of the second section 74 abuts an end surface 73b of the finger 73 of the first section 72 to define a first length of the spinal rod 70. In the second configuration, the tip 73a of the finger 73 of the first section 72 is spaced-apart from the end surface 75b of the finger 75 of the second section 74 and the tip 75a of the finger 75 of the second section 74 is spaced-apart from the end surface 73b of the finger 73 of the first section 72 to define a second length of the spinal rod 70 greater than its first length. In the third configuration, the tip 73a of the finger 73 of the first section 72 is spaced-apart from the end surface 75b of the finger 75 of the second section 74 and the tip 75a of the finger 75 of the second section 74 is spaced-apart from the end surface 73b of the finger 73 of the first section 72 until a rib 76 of the finger 73 abuts a rib 76 of the finger 75 to define a third length of the spinal rod 70 greater than its second length. The abutment of ribs 76 (FIG. 6) limits the maximum length of the spinal rod 70. It is contemplated that the coupling members 78 may provide resistance to the sliding of the fingers 73, 75 against one another.

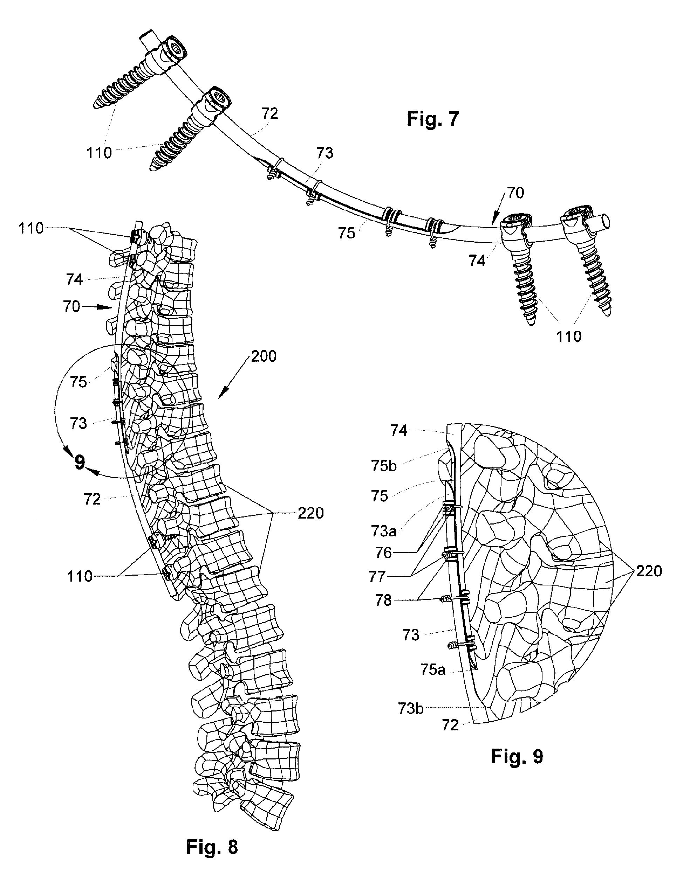

Continuing to refer to FIGS. 3-5, the spinal rod 70 defines a linear longitudinal axis. With reference to FIGS. 7 and 8, it is with the scope of this disclosure that the spinal rod 70 may define a radius of curvature that is concave (FIG. 7) relative to a spine or convex (FIG. 8) relative to a spine.

With reference to FIGS. 8 and 9, the spinal rod 70 is secured to vertebral bodies of a spine 200 of a patient with pedicle screws 110 in accordance with the present disclosure. To secure the spinal rod 70 to the spine 200 of a patient, pedicle screws 110 are secured to vertebral bodies 220 of the spine 200 of a patient by known means. The length of the spinal rod 70 is adjusted such that the first section 72 is receivable in the head of one or more pedicle screws 110 secured to a first vertebral body 220 and the second section 74 is receivable in the head of one or more pedicle screws 110 secured to a different vertebral body 220 with the fingers 73, 75 are positionable between the pedicle screws 110. With the length of the spinal rod 70 adjusted to a desired length, the first section 72 is secured to the head of a pedicle screw 110 and the second section 74 is secured to the head of another pedicle screw 110 with the fingers 73, 75 positioned between the pedicle screws 110.

It is within the scope of this disclosure that other the components and/or instruments (e.g., rod reducers, rod benders, bone screws, etc.) may be used to secure the spinal rod into the head of the pedicle screws 110. Examples of such other components and instruments are disclosed in U.S. Patent Application Serial No. 13/636,416, filed on November 8, 2012, and published as U.S. Patent Publication No. 2013/0144342 on June 6, 2013, the entire contents of which are incorporated by reference herein. In addition, the head of the pedicle screws 110 may secure to the spinal rods with a set screw configuration (FIG. 7) or with a taper lock configuration (FIG. 8).

It is within the scope of this disclosure that the spinal rod 70 may be secured to the vertebral bodies 22 (FIG. 1) of the spine model 20 in a similar manner to the spinal rod 60 being secured to the spine 200 of a patient as detailed above. This may allow a clinician to practice methods of placing spine rod 70 on a spine of a patient before performing such a procedure on a patient. The spine model 20 may be adjusted such that the spine model 20 is similar to a spine of a particular patient. In addition, the spine model 20 may be manipulated with the spinal rod 70 secured to the spine model 20, as detailed above, such that a clinician may observe the spinal rod 70 interacting with the spine model 20 as the spine model 20 simulates a growing spine or a spine responding to a patient's movements. As the spine model 20 simulates a growing spine, it is contemplated that the spinal rod 70 may transition between the first, second, and third configurations.

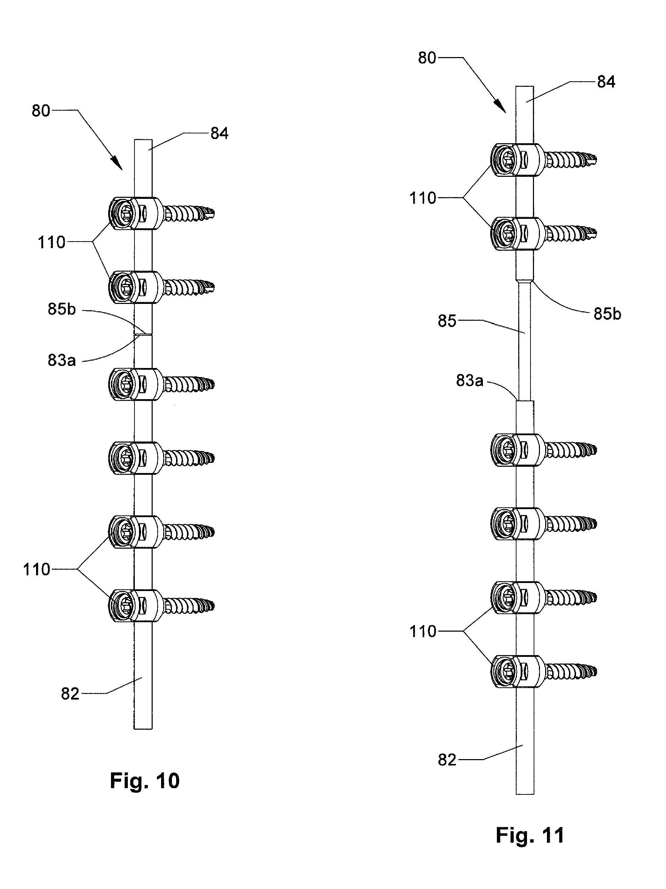

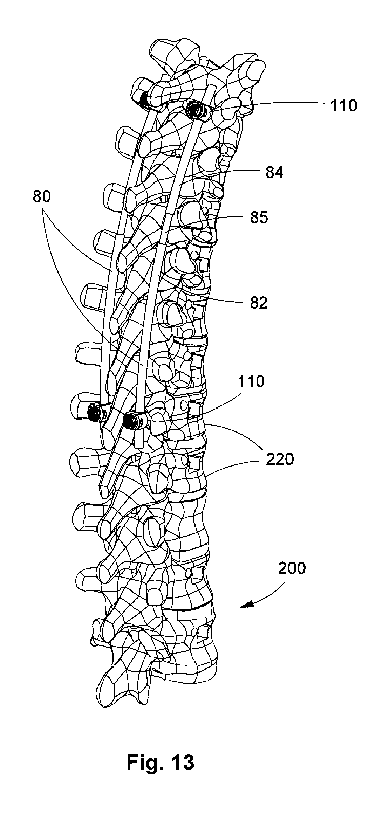

Referring to FIGS. 10-13, another adjustable spinal rod 80 is provided in accordance with the present disclosure and includes a first section 82 and a second section 84. Each of the first and second sections 82, 84 may be secured to vertebral bodies (e.g., vertebral bodies 22 of a spine model 20 (FIG. 1) or vertebral bodies 220 of a spine 200 (FIG. 13)). The first section 82 defines a first diameter D₁ and the second section 84 defines a third diameter D₃ substantially equal to the first diameter D₁. The first section 82 defines a blind hole 83 in an end facing the second section 84. The blind hole 83 is disposed about the longitudinal axis of the spinal rod 80 and defines a second diameter D₂ less that the first and third diameters D₁, D₃. The second section 84 includes a shaft 85 extending towards the first section 82 that defines a fourth diameter D₄ that is less than the second diameter D₂ of the blind hole 83 such that the shaft 85 is slidably receivable within the blind hole 83 of the first section 82. It is contemplated that the fourth diameter D₄ is substantially equal to the second diameter D₂ such that the shaft 85 provides resistance when slid within the blind hole 83. The blind hole 83 defines a depth along the longitudinal axis of the spinal rod 80 between a tip 83a of the first section 82 and an end surface 83b within the blind hole 83. The shaft 85 has a shaft length along the longitudinal axis of the spinal rod 80 between a tip 85a and an end surface 85b.

In a first configuration of the spinal rod 80 (FIG. 10), the tip 83a of the first section 82 abuts the end surface 85b of the second section 84 such that the spinal rod 80 defines a first length. In a second configuration of the spinal rod 80 (FIG. 11), the tip 83a of the first section 82 is spaced-apart from the end surface 85b of the second section 84 such that the spinal rod 80 defines a second length greater than its first length. It will be appreciated that in the second configuration of the spinal rod 80, the tip 85a is positioned within the blind hole 83 of the first section 82.

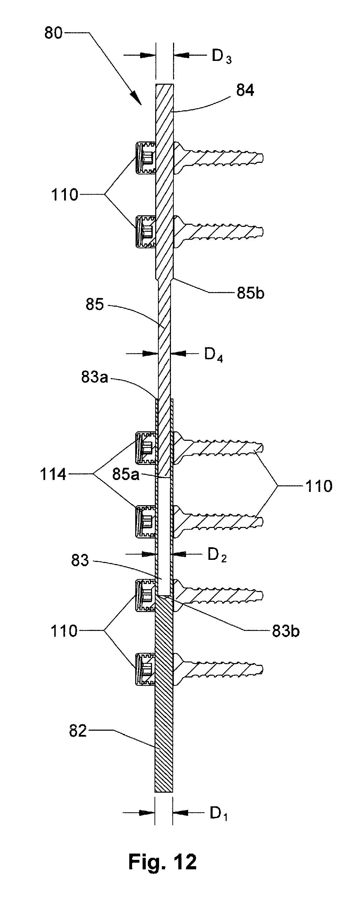

With particular reference to FIG. 12, it is contemplated that the head 114 of one or more of the pedicle screws 110 may compress the walls of the blind hole 83 into engagement with the shaft 85 to longitudinally fix the first and second sections 82, 84 relative to one another. It is contemplated that walls of the blind hole 83 may define ratchets (not shown) and the outer surface of the shaft 85 may include teeth (not shown) that engage the ratchets to longitudinally fix or limit the length of the spinal rod 80. Additionally or alternatively, it is contemplated that the tip 85a of the shaft 85 may define a diameter greater than the diameter of the shaft 85 and the tip 83a of the of the first section 82 may extend into the blind hole 83 such that at the tip 83a of the first section 82 the blind hole 83 has a diameter greater than the diameter of the shaft 85 and less than the diameter of the tip 85a of the shaft 85 to limit the length of the spinal rod 80.

It is within the scope of this disclosure that both the first and second sections 82, 84 of the spinal rod define blind holes 83. Each of the blind holes 83 slidably receiving a shaft 85 that is separate from the first and second sections 82, 84.

As shown in FIGS. 10-12, the spinal rod 80 is linear along its longitudinal axis. With reference to FIG. 13, the spinal rod 80 defines a radius of curvature similar to the spinal rod 70 detailed above. In addition, the spinal rod 80 is securable to vertebral bodies (e.g., vertebral bodies 22 of a spine model 20 (FIG. 1) or vertebral bodies 220 of a spine 200 (FIG. 13)) in a similar manner to spinal rod 70 as detailed above.

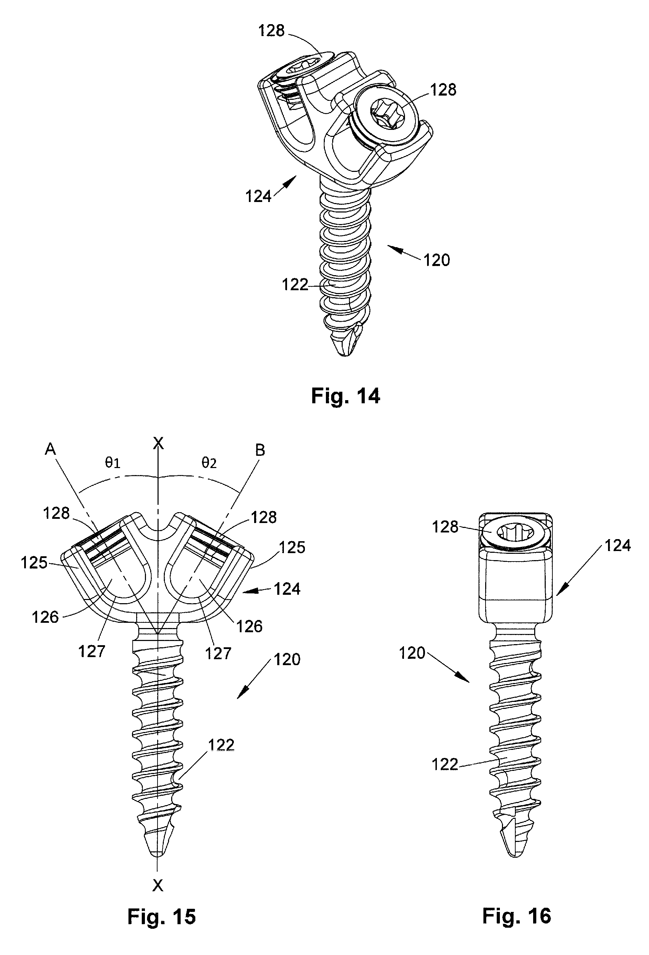

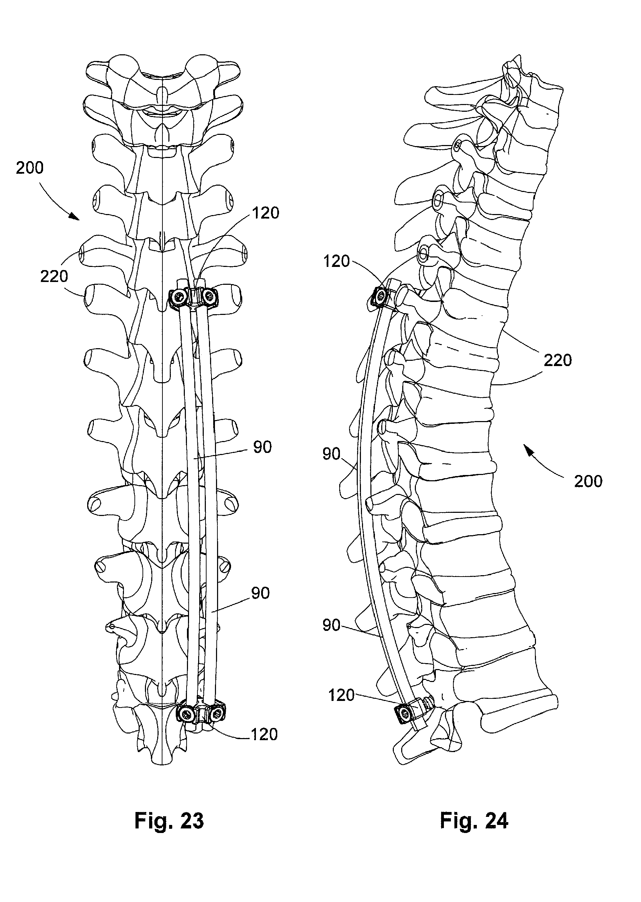

Referring to FIGS. 14-16, a pedicle screw 120 is provided in accordance with the present disclosure and includes a shank 122 and a head 124. The shank 122 is configured to penetrate and secure the pedicle screw 120 to a vertebral body (e.g., vertebral bodies 22 of a spine model 20 (FIG. 1) or vertebral bodies 220 of a spine 200 (FIG. 23)). The head 124 of the pedicle screw 120 is configured to secure two spinal rods (e.g., spinal rods 60, 70, 80, 90) to the pedicle screw 120. The head 124 includes two saddles 125 that each define a U-shaped channel 126. Each saddle 125 is configured to secure a spinal rod within a respective channel 126. Each channel 126 has a bottom surface 127 that is sized and shaped to receive a spinal rod. Each saddle 125 is threaded to receive a set screw 128 opposite the bottom surface 127 to secure a spinal rod within the channel 126 as detailed below.

With particular reference to FIG. 15, the saddles 125 define axes A and B that are offset from a longitudinal axis X-X defined by the shank 122 of the pedicle screw 120. The axis A is offset from the axis X-X by a first angle θ₁ and the axis B is offset from the axis X-X by a second angle θ₂. As shown, the first and second angles θ₁, θ₂ are substantially equal to one another and are approximately 30°. In addition, the first and second angles θ₁, θ₂ are disposed in the same plane and in different directions from the axis X-X. It is within the scope of this disclosure for the first and second angles θ₁, θ₂ to be disposed in different planes from one another. It is contemplated that the first and second angles θ₁, θ₂ may be the same or different from one another and be in a range of about 0° to about 90°. Some non-limiting examples of combinations of the first and second angles θ₁, θ₂ are 0° and 90°, 10° and 90°, 15° and 90°, 20° and 90°, 30° and 90°, 45° and 90°, 0° and 45°, 20° and 20°, and 45° and 45°. It is also contemplated that the first and second angles θ₁, θ₂ may be in the same direction from the axis X-X. In addition, it is within the scope of this disclosure that the head 124 may pivot or rotate relative to the shank 122.

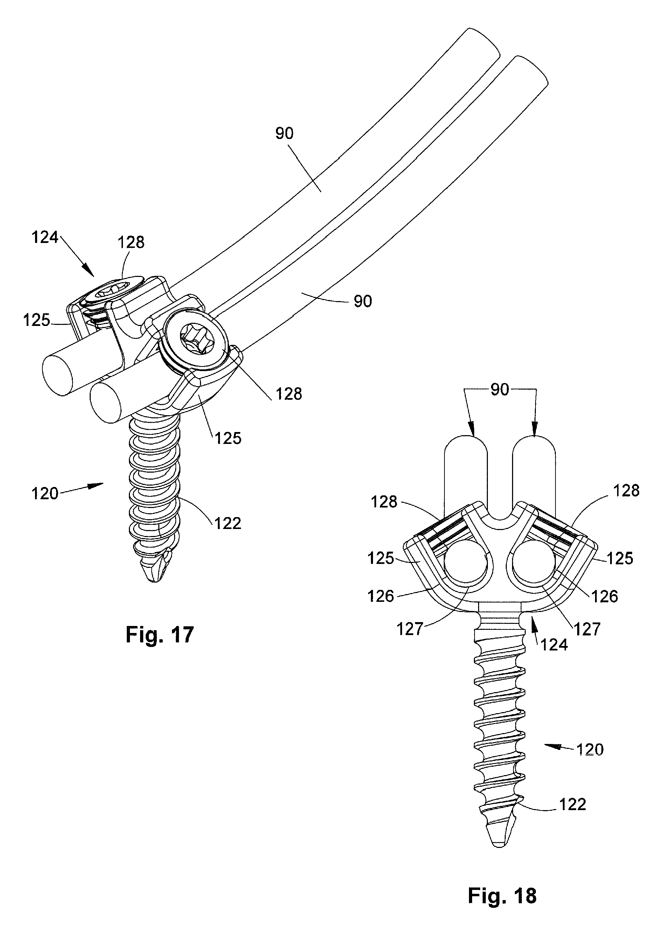

Referring to FIGS. 17 and 18, spinal rods 90 are secured to the head 124 of the pedicle screw 120 in accordance with the present disclosure. To secure the spinal rods 90 to the pedicle screw 120, the pedicle screw 120 is first secured to a vertebral body as detailed above with respect to pedicle screw 110. When the pedicle screw 120 is secured to a vertebral body, one of the spinal rods 90 is positioned within the channel 126 of one of the saddles 125. A set screw 128 is threaded into the saddle 125 until the set screw 128 engages the spinal rod 90 to engage the spinal rod 90 with the bottom surface 127 of the channel 126. The set screw 128 is then torqued to secure the spinal rod 90 to the head 124 of the pedicle screw. This is repeated for the other spinal rod and the other saddle 125. It is contemplated that one of a first set screw 128 may be partially torqued to partially secure a first spinal rod 90 in one of the saddles 125 while the second spinal rod 90 is positioned within and secured within the other saddle 125 before fully torquing the first set screw 128 to fully secure the first spinal rod 90 in its saddle 125.

While shown with respect to spinal rod 90, it is contemplated that one or more of spinal rods 60, 70, or 80 may also be secured within the head 124 of pedicle screw 120. In addition, while each saddle 125 is shown with a set screw 128 to secure a spinal rod within the channel 126, it is within the scope of this disclosure that each saddle 125 may include a taper lock configuration (FIG. 8) to secure a spinal rod within the channel 126. Such a taper lock configuration is disclosed in U.S. Patent No. 7,988,694, the entire contents of which are hereby incorporated by reference.

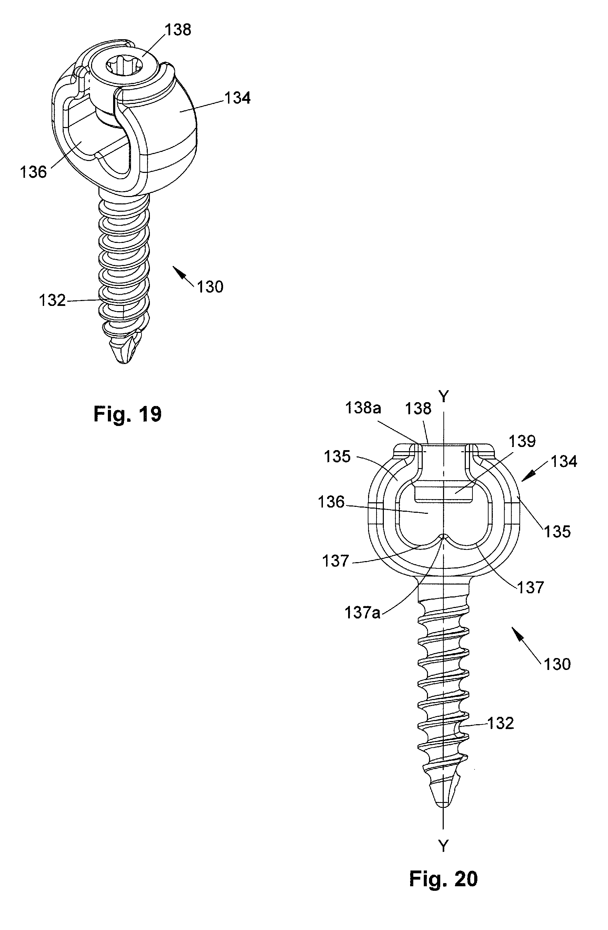

Referring to FIGS. 19 and 20, another pedicle screw 130 is provided in accordance with the present disclosure and includes a shank 132 and a head 134. The head 134 includes sidewalls 135 that define a channel 136 therebetween. The channel 136 has bottom surfaces 137 that are shaped to each receive a spinal rod as detailed below. The bottom surfaces 137 are separated by a divider 137a that extends upward between the bottom surfaces 137. The divider 137a is aligned with an axis Y-Y of the shank 132. The sidewalls 135 define an opening 138a opposing the bottom surfaces 137. The sidewalls 135 are biased towards one another at the opening 138a and the opening 138a is threaded to receive a threaded set screw 138. As the set screw 138 is threaded into the opening 138a, the set screw 138 urges the sidewalls 135 apart. The set screw 138 includes a lower extension 139 to engage and secure spinal rods 90 within the channel 136 as detailed below. It is within the scope of this disclosure that the head 134 may pivot or rotate relative to the shank 132.

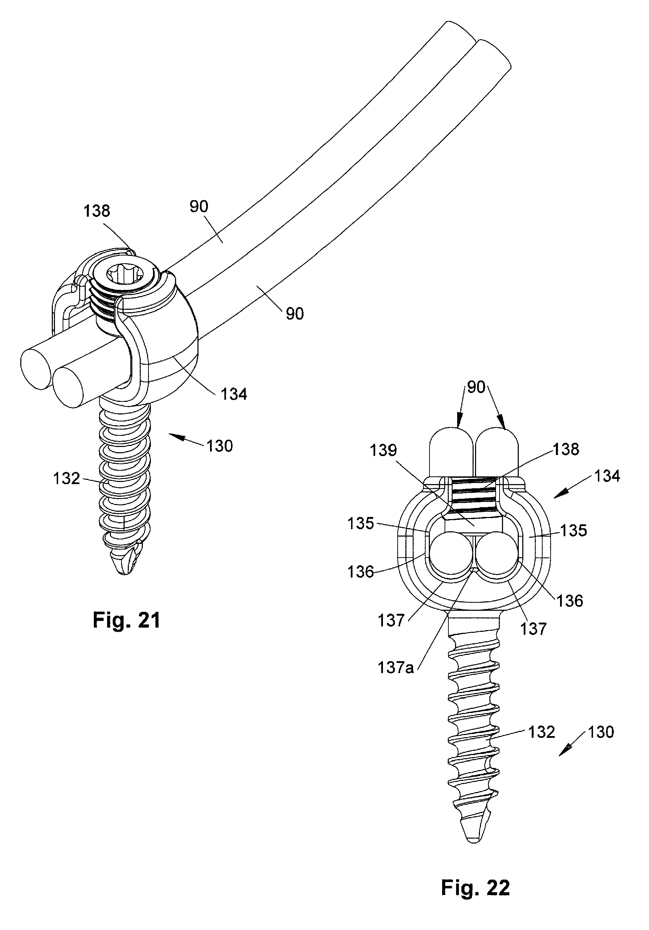

With reference to FIGS. 21 and 22, spinal rods 90 are secured to the head 134 of the pedicle screw 130 in accordance with the present disclosure. To secure the spinal rods 90 to the pedicle screw 130, the pedicle screw 130 is first secured to a vertebral body as detailed above with respect to pedicle screw 110. When the pedicle screw 130 is secured to a vertebral body, of a spine or spine model, the spinal rods 90 are positioned within the channel 136. The set screw 138 is threaded into the opening 138a defined by the sidewalls 135 until the lower extension 139 of the set screw 138 engages the spinal rods 90 to move the spinal rods 90 into engagement with the bottom surfaces 137 of the channel 136. The spinal rods 90 may engage the divider 137a to guide each spinal rod 90 into engagement with one of the bottom surfaces 137. The set screw 138 is then torqued to secure the spinal rods 90 to the head 134 of the pedicle screw 130.

It is contemplated that the lower extension 139 of the set screw 138 may have a complimentary shape to the bottom surfaces 137 and include a divider (not shown) to separate the spinal rods 90 within the channel 135 as the set screw 138 is torqued to secure the spinal rods 90 to the head 134 of the pedicle screw 130.

With reference to FIGS. 23 and 24, the pedicle screw 120 is used to secure to spinal rods 90 to a spine 200 of a patient. As shown, the spinal rods 90 have a substantially equal length to one another and extend over the same vertebral bodies 220 of the spine 200. It will be appreciated that the pedicle screw 120 or the pedicle screw 130 may be used with the spine model 20 in a manner similar to that detailed below with respect to the spine 200.

It is also contemplated that spinal rods 90 may be staggered such that one spinal rod 90 is secured to one pair of pedicle screws 120 and a second spinal rod 90 is secured to a second pair of pedicle screws 120 where the first and second pair of pedicle screws 120 only share one common pedicle screw. In such configurations, the first spinal rod 90 extends over different vertebral bodies 220 than the second spinal rod 90.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and is within the scope of the appended claims. Therefore, the above description should not be construed as limiting, but merely as exemplifications of particular embodiments. Those skilled in the art will envision other modifications within the scope of the claims appended hereto.

## Claims

1. An adjustable spinal rod comprising:
a first section having a first base portion and a first coupling portion extending from the first base portion; and
a second section having a second base portion and a second coupling portion extending from the second base portion, the second coupling portion axially aligned and slidably engaged with the first coupling portion,
wherein the adjustable spinal rod has a first configuration defining a first length and a second configuration defining a second length different from the first length.

2. The adjustable spinal rod according to claim 1, wherein the first base portion defines a circular cross-section and the first coupling portion includes a first finger that defines a semi-circular cross-section, wherein the second base portion defines a circular cross-section and the second coupling portion includes a second finger that defines a semi-circular cross-section, and wherein the first and second fingers slidably engaged with one another.

3. The adjustable spinal rod according to claim 2, further comprising a first coupling member disposed about the first and second fingers to slidingly couple the first and second sections together.

4. The adjustable spinal rod according to claim 3, wherein the first finger includes a pair of ribs that define a first groove therebetween.

5. The adjustable spinal rod according to claim 4, wherein a portion of the first coupling member is positionable in the first groove about the first and second fingers.

6. The adjustable spinal rod according to claim 5, wherein the second finger includes a pair of ribs that define a second groove therebetween, a portion of the second coupling member positionable within the second groove about the first and second fingers.

7. The adjustable spinal rod according to any one of claims 1-5, wherein the first section has a first diameter and the first coupling portion defines a blind hole about a longitudinal axis of the first section, the blind hole having a second diameter, and wherein the second base portion has a diameter equal to the first diameter of the first base portion and the second coupling portion having a third diameter less than the second diameter of the blind hole, the second coupling portion slidably receivable within the blind hole of the first coupling portion.

8. The adjustable spinal rod according to claim 7, further comprising a coupling member disposable about the first coupling portion to fix the first and second coupling portions relative to one another.

9. The adjustable spinal rod according to claim 8, wherein the coupling member is disposed about the first and second coupling portions.

10. The adjustable spinal rod according to any one of claims 1-9, wherein the first and second sections define a curved longitudinal axis of the adjustable spinal rod.

11. A spinal construct comprising:
a first adjustable spinal rod according to any one of claims 1-10;
a second adjustable spinal rod according to any one of claims 1-10; and
a pedicle screw including a head and a shank, the shank configured to secure the pedicle screw to a bony element, the head disposed at one end of the shank and receiving the first base portion of the first adjustable spinal rod and the first base portion of the second adjustable spinal rod to fix the first and second adjustable spinal rods relative to one another.

12. The spinal construct according to claim 11, wherein the head defines a first saddle and a second saddle, the first saddle receiving the first base portion of the first adjustable spinal rod to fix the first adjustable spinal rod to the pedicle screw, the second saddle receiving the first base portion of the second adjustable spinal rod to fix the second adjustable spinal rod to the pedicle screw, the first and second saddles configured to independently fix the first and second adjustable spinal rods to the pedicle screw.

13. The spinal construct according to claim 12, wherein the shank defines a longitudinal shank axis, wherein the first saddle defines a first saddle axis offset from the shank axis by a first angle, and wherein the second saddle defines a second saddle axis offset from the shank axis by a second angle, the first and second angles defined in a common plane.

14. The spinal construct according to claim 13, wherein the first and second angles are defined in different directions from the shank axis.

15. The spinal construct according to claim 11, wherein the head has sidewalls defining a channel, the channel receiving the first base portion the first adjustable spinal rod and the first base portion of the second adjustable spinal rod.
